# DEMANDE DE BREVET EUROPEEN

(11) **EP 4 098 249 A1**
(43) Date de publication de la demande: **07.12.2022**
(21) Numéro de dépôt: 22177122.3
(22) Date de dépôt: 03.06.2022
(51) Int. Cl.: A61K 9/08, A61K 31/135, A61K 47/10, A61K 47/38, A61K 47/18

(54) **UTILISATION DE L'AMITRIPTYLINE ET/OU L'UN DE SES SELS PHARMACEUTIQUEMENT ACCEPTABLE COMME AGENT CONSERVATEUR**

(30) Priorité: 03.06.2021 FR 2105871
(71) Demandeur: Algotherapeutix, 92150 Suresnes (FR)
(72) Inventeur: LALLEMAND, Frédéric, 78120 Rambouillet (FR); PICAUT, Philippe, 91370 Verrières le Buisson (FR)
(74) Mandataire: Casalonga

(57) **Abrégé**

La présente invention concerne l'utilisation de l'amitriptyline et/ou l'un de ses sels pharmaceutiquement acceptables comme agent conservateur dans une composition pharmaceutique.

## Description

La présente invention concerne l'utilisation de l'amitriptyline et/ou l'un de ses sels pharmaceutiquement acceptables comme agent conservateur dans une composition pharmaceutique.

Les compositions pharmaceutiques et les substances qu'elles contiennent sont soumises à des normes importantes, notamment en termes de présence de microorganismes, tels que des bactéries, levures ou champignons, afin d'éviter les risques d'infections pour l'utilisateur et la détérioration des compositions pharmaceutiques.

En Europe, le Chapitre 5.1.4 « Qualité microbiologique des préparations pharmaceutiques » de la Pharmacopée Européenne indique que les formulations administrées par voie topique n'ont pas besoin d'être stériles. Néanmoins, elles ne doivent pas comprendre plus de cent microorganismes par gramme de composition et elles ne doivent pas comprendre de *Staphylococcus aureus*, ni *de Pseudomonas aeruginosa.*

Afin de vérifier leur bonne conformité avec ces normes avant leur mise sur le marché, les compositions doivent être soumises à des tests tels que ceux spécifiés par les monographies 2.6.12 et 2.6.13 obligeant un dénombrement des germes et microorganismes et fixant un seuil limite à ne pas dépasser.

Pour respecter ces normes et maintenir une population en microorganismes aussi basse, il est souvent nécessaire d'incorporer dans les compositions pharmaceutiques des conservateurs ou agents antimicrobiens. Le Chapitre 5.1.3 « Efficacité de la conservation antimicrobienne » de la Pharmacopée Européenne défini clairement comment doit être évaluer l'efficacité d'un conservateur antimicrobien dans un produit pharmaceutique.

De telles normes et de tels tests sont également en vigueur aux Etats Unis (spécifications USP, Chapitre 51).

Parmi les conservateurs couramment utilisés dans les compositions pharmaceutiques, on retrouve notamment les parabènes, tel que le méthyl parabène. Or ces composés sont suspectés d'interagir avec les récepteurs de certaines hormones et donc d'avoir un rôle de perturbateurs endocriniens.

Il existe donc un réel besoin de mettre à disposition de nouveaux conservateurs antimicrobiens utilisables dans les compositions pharmaceutiques, en particulier les compositions pharmaceutiques topiques.

Il a été découvert de manière surprenante que l'amitriptyline et/ou l'un de ses sels pharmaceutiquement acceptables permettait d'obtenir un effet antimicrobien équivalent à un conservateur de type parabènes dans des compositions pharmaceutiques.

La présente invention a donc pour objet l'utilisation de l'amitriptyline et/ou l'un de ses sels pharmaceutiquement acceptables comme agent conservateur dans une composition pharmaceutique.

L'utilisation de l'amitriptyline et/ou l'un de ses sels permet de formuler des compositions pharmaceutiques avec une quantité faible, voire nulle, de conservateur de type parabènes. Une telle utilisation permet donc de limiter la présence de perturbateurs endocriniens potentiels ou de conservateurs présentant des risques de provoquer des allergies ou des irritations.

La présente invention porte également sur un procédé pour la conservation d'une composition pharmaceutique, de préférence pour inhiber la croissance de microorganismes tels que des bactéries, levures et/ou champignons dans une composition pharmaceutique, comprenant l'ajout dans ladite composition pharmaceutique, de l'amitriptyline et/ou l'un de ses sels pharmaceutiquement acceptable.

D'autres objets, caractéristiques, aspects et avantages de l'invention apparaîtront encore plus clairement à la lecture de la description et de l'exemple qui suit.

Dans la présente description, et à moins d'une indication contraire :
- l'expression "au moins un" est équivalente à l'expression "un ou plusieurs" et peut y être substituée ;
- l'expression "compris entre...et..." est équivalente à l'expression "allant de...à..." et peut y être substituée, et sous-entend que les bornes sont incluses ;
- l'expression « polyoxyalkyléné » correspond, au sens de l'invention, à un motif -(O-alkyle)ₙ-, où n est un nombre entier variant de 2 à 200, de préférence de 2 à 40, plus préférentiellement de 2 à 20 ;
- l'expression « polyoxyéthyléné » correspond, au sens de l'invention, à un motif -(O-CH₂CH₂)ₙ-, où n est un nombre entier variant de 2 à 200, de préférence de 2 à 40, plus préférentiellement de 2 à 20 ;
- l'expression « la composition est exempte d'un composé » signifie, au sens de la présente invention, que la composition contient moins de 2% en poids dudit composé, de préférence moins de 1% en poids dudit composé, plus préférentiellement moins de 0,5% en poids dudit composé, mieux moins de 0,1% en poids, mieux encore moins inférieure à 0,05% en poids, par rapport au poids total de la composition, et mieux encore que la composition ne contient pas ledit composé. Dans ce type de composition, tout « composé » présent n'est pas ajouté lors de la préparation de la composition mais correspond aux traces de « composé » résiduel apporté par les ingrédients mélangés.

### Amitriptyline et ses sels

L'utilisation selon la présente invention met en œuvre au moins de l'amitriptyline et/ou l'un de ses sels pharmaceutiquement acceptables dans une composition pharmaceutique.

L'amitriptyline est de formule (I) suivante :

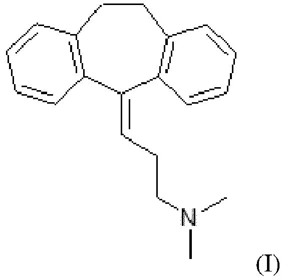

Dans le cadre de la présente invention, on entend par « *sels d'amitriptyline pharmaceutiquement acceptables* », les sels compatibles avec une composition pharmaceutique c'est-à-dire destinée à être administrée à des humains. En particulier, on entend par sel d'amitriptyline pharmaceutiquement acceptable les hydrates, les solvates, les sels d'acides tel que les chlorhydrates et les clathrates de l'amitriptyline.

Comme sel tout particulièrement préféré de l'amitriptyline, on utilisera le chlorhydrate d'amitriptyline.

De préférence, la teneur totale en amitriptyline et/ou en l'un de ses sels pharmaceutiquement acceptable est comprise entre 1 et 25% en poids, plus préférentiellement entre 5 et 20% en poids, plus préférentiellement encore entre 5 et 15% en poids, mieux entre 10 et 15% en poids, par rapport au poids total de la composition.

Plus préférentiellement, la teneur totale en chlorhydrate d'amitriptyline est comprise entre 1 et 25% en poids, plus préférentiellement encore entre 5 et 20% en poids, encore mieux entre 5 et 15% en poids, mieux entre 10 et 15% en poids, par rapport au poids total de la composition.

La présence d'amitriptyline et/ou l'un de ses sels pharmaceutiquement acceptables dans la composition pharmaceutique selon l'invention permet d'obtenir une composition conforme aux critères de la Pharmacopée Européenne et américaine, notamment en matière de présence de microorganismes dans la composition. En particulier, la présence d'amitriptyline et/ou l'un de ses sels pharmaceutiquement acceptables permet de limiter, voire d'empêcher, la croissance de microorganismes tels que *Pseudomonas aeruginosa*, *Staphylococcus aureus*, *Escherichia coli*, *Candida albicans, Aspergillus brasiliensis.*

La composition peut éventuellement comprendre en outre au moins un agent conservateur additionnel, différent de l'amitriptyline et de ses sels, tel que le méthyl parabène. Dans ce cas, la teneur en agent(s) conservateur(s) additionnel(s) est de préférence inférieure ou égale à 10% en poids, plus préférentiellement inférieure ou égale à 5% en poids, plus préférentiellement encore inférieure ou égale à 1% en poids, mieux inférieure ou égale à 0,5% en poids, par rapport au poids total de la composition.

La composition selon l'invention peut éventuellement comprendre en outre un ou plusieurs agents conservateurs additionnels, différentes de l'amitriptyline et de ses sels, tels que le méthyl parabène, l'éthyl parabène, le propyl parabène, le butyl parabène, le chlorure de benzalkoniumn, le sorbate de potassium, le phenoxyéthanol, la chlorhexidine, l'alcool benzylique, l'acide borique, le butylène glycol, le cétrimide, le chlorobutanol, le benzoate de sodium

Dans un mode de réalisation préféré de l'invention, la composition selon l'invention est exempte d'agent conservateur additionnel. En d'autres mots, l'amitriptyline et/ou l'un de ses sels pharmaceutiquement acceptables est(sont) le(s) unique agent(s)conservateur(s) de la composition selon l'invention. En particulier, de préférence, la composition selon l'invention est exempte de parabène, tel que le méthyl parabène, éthyl parabène, propyl parabène, butyl parabène, chlorure de benzalkoniumn, sorbate de potassium, de phenoxyéthanol, chlorhexidine, alcool benzylique, acide borique, butylène glycol, cétrimide, chlorobutanol, benzoate de sodium.

### Eau

La composition selon la présente invention peut comprendre de l'eau.

De préférence, la teneur totale en eau est supérieure ou égale à 75% en poids, plus préférentiellement comprise entre 75 et 95% en poids ; plus préférentiellement encore entre 75 et 90% en poids, par rapport au poids total de la composition.

### Polyols

De préférence, la composition selon la présente invention peut comprendre en outre au moins un polyol en C₂-C₈.

Par « polyol en C₂-C₈ » au sens de la présente invention, on entend un composé organique constitué d'une chaîne hydrocarbonée en C₂-C₈, éventuellement interrompue par un ou plusieurs atomes d'oxygène, et porteuse d'au moins deux groupements hydroxyles libres (-OH) portés par des atomes de carbone différents, ce composé pouvant être cyclique ou acyclique, linéaire ou ramifié, saturé ou insaturé, et à l'état liquide à température ambiante (25°C) et à pression atmosphérique (soit 1,013.10⁵ Pa).

De préférence, le ou les polyols en C₂-C₈ selon l'invention sont acycliques et non-aromatiques.

Les polyols en C₂-C₈ selon l'invention comprennent dans leur structure de 2 à 8 atomes de carbone, de préférence de 2 à 6 atomes de carbone, plus préférentiellement de 2 à 5 atomes de carbone.

Plus particulièrement, le ou les polyols utilisables selon l'invention comprennent de 2 à 10 groupements hydroxy, plus préférentiellement de 2 à 5 groupements hydroxy, plus préférentiellement encore de 2 à 3 groupements hydroxy.

De manière préférée, le ou lesdits polyols en C₂-C₈ utilisables selon l'invention sont choisis parmi les polyols en C₃-C₆, l'éthylène glycol, et leurs mélanges.

De préférence, les polyols sont différents des agent(s) conservateur(s) additionnel(s) éventuellement présent(s) dans la composition.

Selon un mode de réalisation préféré de l'invention, le ou lesdits polyols en C₂-C₈ utilisables selon l'invention sont choisis parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et un mélange de ces composés ; plus préférentiellement la composition comprend au moins le propylène glycol.

De préférence, lorsque la composition selon l'invention comprend au moins un polyol en C₂-C₈, la teneur totale en polyol(s) en C₂-C₈ est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition.

De préférence, lorsque la composition selon l'invention comprend au moins un polyol en C₂-C₈, la teneur totale en propylène glycol est comprise entre 0,1 et 15% en poids, plus préférentiellement entre 0,5 et 10% en poids, plus préférentiellement encore entre 1 et 6% en poids, et encore mieux entre 3 et 6% en poids, par rapport au poids total de la composition.

### Les agents épaississants

La composition selon l'invention peut comprendre en outre au moins un agent épaississant.

Plus préférentiellement, le ou les agents épaississants sont des polymères épaississants.

Par « polymère épaississant », on entend selon la présente invention des polymères qui augmentent, par leur présence à une concentration de 0,05% en poids, la viscosité des compositions cosmétiques dans lesquelles ils sont introduits d'au moins 20 cps (20 mPa.s), de préférence d'au moins 50 cps (50 mPa.s), à température ambiante (25°C), à pression atmosphérique et à un taux de cisaillement de 1s⁻¹ (la viscosité peut être mesurée à l'aide d'un viscosimètre cône/plan, Rhéomètre Haake R600 ou analogue).

À titre d'exemple, on peut citer comme agent épaississant : les homopolymères ou copolymères d'acide acrylique ou méthacryliques réticulés, homopolymères réticulés d'acide 2-acrylamido-2-méthyl-propane sulfonique et leurs copolymères réticulés d'acrylamide, les homopolymères d'acrylate d'ammonium ou les copolymères d'acrylate d'ammonium et d'acrylamide, les polymères cellulosiques, et leurs mélanges.

Parmi les homopolymères d'acide acrylique réticulés, on peut citer ceux réticulés par un éther allylique d'alcool de la série du sucre, comme par exemple les produits vendus sous les noms CARBOPOLS 980, 981, 954, 2984 et 5984 par la société NOVEON ou les produits vendus sous les noms SYNTHALEN M et SYNTHALEN K par la société 3 VSA. Ces polymères ont pour dénomination INCI Carbomer. Les polymères épaississants peuvent être aussi des copolymères d'acide (méth)acryliques réticulés tels que le polymère vendu sous la dénomination AQUA SF1 par la société NOVEON.

Plus préférentiellement encore, la composition selon l'invention comprend en outre au moins un polymère cellulosique.

Par polymère « cellulosique », on entend selon l'invention tout composé polysaccharidique, substitué ou non, possédant dans sa structure des enchaînements de résidus glucose unis par des liaisons β-1,4 ; outre les celluloses non substituées, les dérivés de celluloses peuvent être anioniques, cationiques, amphotères ou non-ioniques.

Ainsi, les polymères cellulosiques utilisables selon l'invention peuvent être choisis parmi les celluloses non substituées y compris sous une forme microcristalline et les celluloses substituées.

Plus préférentiellement, les polymères cellulosiques utilisables selon l'invention ne comportent pas de chaîne grasse latérale en C₁₀-C₃₀ dans leur structure.

De préférence, le ou les polymères cellulosiques utilisables selon l'invention présentent un poids moléculaire moyen compris entre 5 000 et 1 500 000, plus préférentiellement entre 50 000 et 800 000, plus préférentiellement encore entre 400 000 et 800 000.

Parmi les polymères cellulosiques selon l'invention, on peut distinguer les éthers de celluloses, les esters de celluloses et les esters éthers de celluloses.

Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose...) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulfates d'éthylcellulose.

Parmi les éthers de cellulose non-ioniques, on peut citer les (C₁-C4)alkylcelluloses telles que les méthylcelluloses et les éthylcelluloses (par exemple Ethocel standard 100 Premium de DOW CHEMICAL) ; les (poly)hydroxy(C₁-C4)alkylcelluloses telles que les hydroxyméthylcelluloses, les hydroxyéthylcelluloses (par exemple Natrosol 250 HHR proposé par AQUALON) et les hydroxypropylcelluloses (par exemple Klucel EF d'AQUALON) ; les celluloses mixtes (poly)hydroxy(C₁-C₄)alkyl-(C₁-C₄)alkylcelluloses telles que les hydroxypropyl-méthylcelluloses (par exemple Methocel E4M de DOW CHEMICAL), les hydroxyéthyl-méthylcelluloses, les hydroxyéthyl-éthylcelluloses (par exemple Bermocoll E 481 FQ d'AKZO NOBEL) et les hydroxybutyl-méthylcelluloses.

Parmi les éthers de cellulose anioniques, on peut citer les (poly)carboxy(C₁-C4)alkylcelluloses et leurs sels. A titre d'exemple, on peut citer les carboxyméthylcelluloses, les carboxyméthylméthylcelluloses (par exemple Blanose 7M de la société AQUALON) et les carboxyméthylhydroxyéthylcelluloses et leurs sels de sodium.

Parmi les éthers de cellulose cationiques, on peut citer les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les (poly)hydroxy(C₁-C₄)alkyl celluloses, comme les hydroxyméthyl-, hydroxyéthyl- ou hydroxypropyl celluloses greffées notamment avec un sel de méthacryloyléthyl-triméthylammonium, de méthacrylmidopropyl-triméthylammonium, de diméthyl-diallylammonium. Les produits commercialisés répondant à cette définition sont plus particulièrement les produits vendus sous la dénomination « Celquat^{®} L 200 » et « Celquat^{®} H 100 » par la Société National Starch.

Selon un mode de réalisation préféré de l'invention, le ou les polymères cellulosiques sont choisis parmi les polymères cellulosiques ne comportant pas de chaîne grasse latérale en C₁₀-C₃₀ dans leur structure ; plus préférentiellement parmi les éthers de cellulose ; plus préférentiellement encore parmi les éthers de cellulose non-ioniques ; encore mieux parmi (a) les (C₁-C₄)alkylcelluloses telles que les méthylcelluloses et les éthylcelluloses, (b) les (poly)hydroxy(C₁-C₄)alkylcelluloses telles que les hydroxyméthylcelluloses, les hydroxyéthylcelluloses et les hydroxypropylcelluloses, (c) les celluloses mixtes (poly)hydroxy(C₁-C₄)alkyl-(C₁-C₄)alkylcelluloses telles que les hydroxypropyl-méthylcelluloses, les hydroxypropyl-éthylcelluloses, les hydroxyéthyl-méthylcelluloses, les hydroxyéthyl-éthylcelluloses et les hydroxybutyl-méthylcelluloses, et (d) leurs mélanges.

Plus préférentiellement, la composition selon l'invention comprend au moins une (poly)hydroxy(C₁-C₄)alkylcellulose telles que les hydroxyméthylcelluloses, les hydroxyéthylcelluloses et les hydroxypropylcelluloses ; encore mieux au moins l'hydroxyéthylcellulose.

De préférence, lorsque la composition selon l'invention comprend au moins un agent épaississant, la teneur totale en agent(s) épaississant(s) est comprise entre 0,1 et 10% en poids, plus préférentiellement entre 0,5 et 5% en poids, plus préférentiellement encore entre 1 et 2,5% en poids, par rapport au poids total de la composition.

De préférence, lorsque la composition selon l'invention comprend au moins un polymère cellulosique, la teneur totale en polymère(s) cellulosique(s) est comprise entre 0,1 et 10% en poids, plus préférentiellement entre 0,5 et 5% en poids, plus préférentiellement encore entre 1 et 2,5% en poids, par rapport au poids total de la composition.

De préférence, lorsque la composition selon l'invention comprend au moins un polymère cellulosique, la teneur totale en (poly)hydroxy(C₁-C₄)alkylcellulose(s) est comprise entre 0,1 et 10% en poids, plus préférentiellement entre 0,5 et 5% en poids, plus préférentiellement encore entre 1 et 2,5% en poids, par rapport au poids total de la composition.

De préférence, lorsque la composition selon l'invention comprend au moins un polymère cellulosique, la teneur totale en hydroxyéthylcellulose est comprise entre 0,1 et 10% en poids, plus préférentiellement entre 0,5 et 5% en poids, plus préférentiellement encore entre 1 et 2,5% en poids, par rapport au poids total de la composition.

### Tensioactifs

La composition selon l'invention peut éventuellement comprendre en outre au moins un tensioactif.

Les tensioactifs utilisables selon l'invention peuvent être choisis parmi les tensioactifs anioniques, les tensioactifs cationiques, les tensioactifs amphotères et/ou zwittérioniques, les tensioactifs non ioniques, et leurs mélanges.

Plus préférentiellement, le ou les tensioactifs utilisables selon l'invention sont choisis parmi les tensioactifs non ioniques.

Les tensioactifs non ioniques utilisables peuvent être choisis parmi les alkyl polyglucosides (APG), les esters de glycérol oxyalkylénés, les esters d'acides gras et de sorbitan éventuellement oxyalkylénés, les esters d'acides gras polyoxyalkylénés (en particulier polyoxyéthylénés et/ou polyoxypropylénés) éventuellement en association avec un ester d'acide gras et de glycérol comme le mélange PEG-100 Stearate / Glyceryl Stearate commercialisé par exemple par la société ICI sous la dénomination Arlacel 165, les esters de sucre oxyalkylénés, et leurs mélanges.

Comme alkylpolyglucosides, on peut citer ceux contenant un groupe alkyle comportant de 6 à 30 atomes de carbone et de préférence de 8 à 16 atomes de carbone, et contenant un groupe glucoside comprenant de préférence 1,2 à 3 unités de glucoside. Les alkylpolyglucosides peuvent être choisis par exemple parmi le decylglucoside (Alkyl-C₉/C₁₁-polyglucoside (1.4)) comme le produit commercialisé sous la dénomination Mydol 10^{®} par la société Kao Chemicals ou le produit commercialisé sous la dénomination Plantacare 2000 UP^{®} par la société Cognis ; le caprylyl/capryl glucoside comme le produit commercialisé sous la dénomination Plantacare KE 3711^{®} par la société Cognis ; le laurylglucoside comme le produit commercialisé sous la dénomination Plantacare 1200 UP^{®} par la société Cognis ; le cocoglucoside comme le produit commercialisé sous la dénomination Plantacare 818 UP^{®} par la société Cognis ; le caprylylglucoside comme le produit commercialisé sous la dénomination Plantacare 810 UP^{®} par la société Cognis ; et leurs mélanges.

Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; les stéarates de glycéryle oxyéthylénés ; et leurs mélanges.

Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.

De préférence, le nombre de moles d'oxyde d'alkylène des tensioactifs non ioniques utilisables selon l'invention varie de 2 à 400 ; plus préférentiellement de 4 à 250.

De préférence, la composition selon l'invention est exempte de tensioactif.

Selon une variante de l'invention, la composition comprend au moins un tensioactif non ionique ; plus préférentiellement un tensioactif non ionique choisi parmi les esters de glycérol polyoxyalkylénés ; plus préférentiellement encore au moins un tensioactif non ionique choisi parmi les esters de glycéryle et d'acides gras hydrogénés et polyoxyéthylénés tel que le PEG-200 hydrogenated glyceryl palmate, les cocoates de glycéryle polyoxyéthylénés tels que le PEG-7 glyceryl cocoate et le PEG-30 glyceryl cocoate, les stéarates de glycéryle polyoxyéthylénés, et leurs mélanges.

Plus préférentiellement encore selon cette variante, la composition comprend au moins un cocoate de glycéryle polyoxyéthyléné.

De préférence, lorsque la composition selon l'invention comprend au moins un tensioactif, la teneur totale en tensioactif(s) est comprise entre 0,1 et 10% en poids, plus préférentiellement entre 0,5 et 5% en poids, plus préférentiellement encore entre 1 et 4% en poids, par rapport au poids total de la composition selon l'invention.

De préférence, lorsque la composition selon l'invention comprend au moins un tensioactif, la teneur totale en tensioactif(s) non ionique(s) est comprise entre 0,1 et 10% en poids, plus préférentiellement entre 0,5 et 5% en poids, plus préférentiellement encore entre 1 et 4% en poids, par rapport au poids total de la composition selon l'invention.

De préférence, lorsque la composition selon l'invention comprend au moins un tensioactif, la teneur totale en ester(s) de glycérol (poly)oxyalkyléné(s) est comprise entre 0,1 et 10% en poids, plus préférentiellement entre 0,5 et 5% en poids, plus préférentiellement encore entre 1 et 4% en poids, par rapport au poids total de la composition selon l'invention.

### Agent anti-oxydant

De préférence, la composition selon l'invention est exempte d'agent anti-oxydant.

Selon une variante de l'invention, la composition comprend en outre au moins un agent anti-oxydant ; plus préférentiellement choisi parmi le tocophérol et ses esters, tels que l'acétate de tocophérol, le gallate de propyle, l'hydroxytoluène butylée (BHT), l'hydroxyanisole butylé (BHA), et leurs mélanges.

### Agent séquestrant

De préférence, la composition selon l'invention est exempte d'agent séquestrant.

Selon une variante de l'invention, la composition comprend en outre au moins un agent séquestrant ; plus préférentiellement choisi parmi (a) l'acide éthylènediamine tétracétique (EDTA) et ses sels tel que le sel disodique d'éthylène diaminetétracétique (Disodium EDTA), (b) les dérivés phosphoniques et leurs sels tels que l'acide hexaméthylène diaminetétra(méthylène phosphonique), l'acide éthylènediamine tétra(méthylène phosphonique), l'acide 1-hydroxyéthylidène-1,1-diphosphonique, l'acide aminotri(méthylènephosphonique), l'acide diéthylène-triamine penta(méthylène phosphonique), (c) les polymères polyaminés tels que les polyalkylène polyamines et leurs dérivés, en particulier la polyéthylèneimine, (d) les dendrimères à activité chélatante, (e) les protéines comme la spermine, la spermidine, la transférine, la ferritine, (f) les acides carboxyliques comme l'acide phytique, l'acide citrique, l'acide malique, l'acide nitrilo-acétique, l'acide fumarique, l'acide tartrique, l'acide succinique, l'acide oxalique, (g) la desferrioxamine mesylate, et leurs mélanges.

La définition d'« agent séquestrant » (également appelé « agent chélatant ») est bien connu de l'homme du métier et fait référence à un composé ou un mélange de composés capable(s) de former un chélate avec un ion métallique. Un chélate est un complexe inorganique dans lequel un composé (l'agent séquestrant ou chélatant) est coordiné à un ion métallique, c'est-à-dire qu'il forme une ou plusieurs liaisons avec l'ion métallique (formation d'un cycle incluant l'ion métallique).

Un agent séquestrant (ou chélatant) comprend généralement au moins deux atomes donneurs d'électrons qui permettent la formation de liaisons avec l'ion métallique.

Selon une autre variante de l'invention, la composition comprend au moins un agent séquestrant et au moins un agent anti-oxydant.

Selon une autre variante de l'invention, la composition est exempte de corps gras, d'agent séquestrant et/ou d'agent anti-oxydant.

### pH

De préférence, le pH de la composition selon l'invention est compris entre 3 et 8, plus préférentiellement entre 4 et 7, et encore mieux entre 5 et 6.

Le pH de ces compositions peut être ajusté à la valeur désirée au moyen d'agents alcalinisants ou d'agents acidifiants habituellement utilisés. Parmi les agents alcalinisants, on peut citer, à titre d'exemples, l'ammoniaque, les alcanolamines, les hydroxydes minéraux ou organiques. Parmi les agents acidifiants, on peut citer, à titre d'exemples, les acides minéraux ou organiques comme l'acide chlorhydrique, l'acide orthophosphorique, les acides carboxyliques comme par exemple l'acide acétique, l'acide tartrique, l'acide citrique, l'acide lactique, les acides sulfoniques.

L'utilisation de l'amitriptyline et/ou l'un de ses sels pharmaceutiquement acceptables comme agent conservateur peut être réalisée dans des compositions pharmaceutiques de différentes formulation, telles qu'un gel aqueux, une solution aqueuse, une émulsion huile dans eau.

Ces compositions peuvent être appliquées par voie topique, en solution buvable, ou encore en injection, etc. De préférence, la composition est appliquée par voie topique, et plus préférentiellement par voie cutanée.

La présente invention porte en outre sur une composition pharmaceutique telle que définie ci-avant comprenant de l'amitriptyline et/ou l'un de ses sels pharmaceutiquement acceptable, de préférence en une teneur totale comprise entre 1 et 25% en poids, plus préférentiellement entre 5 et 20% en poids, plus préférentiellement encore entre 5 et 15% en poids, mieux entre 10 et 15% en poids, par rapport au poids total de la composition, étant entendu que la composition est telle que définie précédemment.

La composition peut éventuellement comprendre en outre au moins un agent conservateur additionnel, différent de l'amitriptyline et de ses sels, tel que le méthyl parabène. Dans ce cas, la teneur en agent(s) conservateur(s) additionnel(s) est de préférence inférieure ou égale à 10% en poids, plus préférentiellement inférieure ou égale à 5% en poids, plus préférentiellement encore inférieure ou égale à 1% en poids, mieux inférieure ou égale à 0,5% en poids, par rapport au poids total de la composition.

Dans un mode de réalisation préféré de l'invention, la composition selon l'invention est exempte d'agent conservateur additionnel. En d'autres mots, l'amitriptyline et/ou l'un de ses sels pharmaceutiquement acceptables est(sont) le(s) unique agent(s) conservateur(s) de la composition selon l'invention. En particulier, de préférence, la composition selon l'invention est exempte de parabène, tel que le méthyl parabène.

La présente invention porte également sur un procédé pour la conservation d'une composition pharmaceutique, de préférence pour inhiber la croissance de microorganismes tels que des bactéries, levures et/ou champignons dans une composition pharmaceutique, comprenant l'ajout dans ladite composition pharmaceutique, de l'amitriptyline et/ou l'un de ses sels pharmaceutiquement acceptable ; de préférence en une teneur totale comprise entre 1 et 25% en poids, plus préférentiellement entre 5 et 20% en poids, plus préférentiellement encore entre 5 et 15% en poids, mieux entre 10 et 15% en poids, par rapport au poids total de la composition, étant entendu que la composition est telle que définie précédemment.

Les exemples suivants illustrent la composition selon l'invention et les avantages de cette composition. Cependant, ils ne représentent en rien une limitation de la présente invention mais illustrent simplement l'invention.

### Exemples

Les compositions A (comparative) et B, C et D (selon l'invention) ont été préparées à partir des ingrédients indiqués dans le tableau ci-après dont les quantités sont exprimées en % en poids :

**[Tableau 1]**

| | A | B | c | D |
|---|---|---|---|---|
| Chlorhydrate d'amitriptyline | 0 | 5 | 10 | 10 |
| Hvdroxvéthvlcellulose | 1 | 1 | 1 | 1 |
| Propylène glycol | 5 | 5 | 5 | 5 |
| Méthvl parabène | 0,1 | 0,1 | 0,1 | 0 |
| Agent de pH | Qsp pH 5,5 ± 0,5 | Qsp pH 5,5 ± 0,5 | Qsp pH 5,5 ± 0,5 | Qsp pH 5,5 ± 0,5 |
| Eau | Qsp 100 | Qsp 100 | Qsp 100 | Qsp 100 |

Les compositions A, C et D ont ensuite été soumises à des tests de conservation antimicrobienne conformément aux réglementations européenne (Chapitre 5.1.3 « Efficacité de la conservation antimicrobienne ») et américaine (USP, Chapitre 51). Les souches microbiennes étudiées sont indiquées dans le tableau suivant :

**[Tableau 2]**

| | Pharmacopée Européenne Chapitre 5.1.3 | USP Chapitre 51 |
|---|---|---|
| *Pseudomonas aeruginosa* | X | X |
| *Staphylococcus aureus* | X | X |
| *Escherichia coli* | | X |
| *Candida albicans* | X | X |
| *Aspergillus brasiliensis* | X | X |

Le dénombrement de la croissance des micro-organismes (ou son absence) est effectué à des intervalles de temps spécifiés à 2, 7, 14 et 28 jours et exprimé en réduction logarithmique par rapport au témoin. Les critères d'acceptation selon les normes européenne et américaine sont présentés dans les tableaux suivants :

**[Tableau 3]**

| Critères de la Pharmacopée Européenne Chapitre 5.1.3 | | T | 2 jours | 7 jours | 14 jours | 28 jours |
|---|---|---|---|---|---|---|
| Miroorganismes | concentration logarithmique de l'inoculum | Critères | Réduction Log (CFU) | | | |
| *P. aeruginosa* | [5-6] | A | ≥ 2 | ≥ 3 | - | NI |
| *S. aureus* | [5-6] | B | - | - | ≥ 3 | NI |
| *C. albicans* | [5-6] | A | - | - | ≥ 2 | NI |
| *A. brasiliensis* | [5-6] | B | - | - | ≥ 1 | NI |

**[Tableau 4]**

| Critères de l'USP Chapitre 51 | | T | 2 jours | 7 jours | 14 jours | 28 jours |
|---|---|---|---|---|---|---|
| Mircoorganimes | concentration logarithmique de l'inoculum | Critères | Réduction Log (CFU) | | | |
| *P. aeruginosa* | [5-6] | Critères USP | - | - | ≥ 2 | NI |
| *S. aureus* | | | | | | |
| *E. coli* | | | | | | |
| *C. albicans* | [5-6] | | - | - | NI | NI |
| *A. brasiliensis* | | | | | | |

| | | | | | | |
|---|---|---|---|---|---|---|
| - T2, 7, 14, 28 jours : résultats exprimés en réduction logarithmique par rapport au contrôle T - NI : Pas d'augmentation du nombre de colonies par rapport au comptage précédent - T : concentration logarithmique en microorganismes de l'inoculum utilisé pour réaliser la contamination | | | | | | |

10g de chacune des compositions A, C et D est inoculé avec 100 µL d'un inoculum de l'un des microorganismes testés comprenant 10⁷ à 10⁸ CFU/ml d'inoculum pour les bactéries et 10⁶ à 10⁷ CFU/ml d'inoculum pour les champignons.

Puis, 1g de composition inoculée par un microorganisme est dilué dans 9mL de solution tampon avant de subir une série de dilutions et d'être distribué dans des boîtes de Petri.

On laisse incuber jusqu'à 28 jours à une température comprise entre 30 et 35°C pour les bactéries et entre 20 et 25°C pour les champignons.

Le nombre de colonies est compté à 2, 7, 14 et 28 jours.

Les résultats sont indiqués dans les tableaux 4 et 5 ci-dessous :

**[Tableau 5]**

| Microorganismes | | *S. aureus* | | | | *P. aeruginosa* | | | |
|---|---|---|---|---|---|---|---|---|---|
| Composition | Temps (i) | 2 | 7 | 14 | 28 | 2 | 7 | 14 | 28 |
| A | | > 4,7 | > 4,7 | > 4,7 | > 4,7 | > 4,7 | > 4,7 | > 4,7 | > 4,7 |
| C | | > 4,7 | > 4,7 | > 4,7 | > 4,7 | > 4,7 | > 4,7 | > 4,7 | > 4,7 |
| D | | > 4,7 | > 4,7 | > 4,7 | > 4,7 | > 4,7 | > 4,7 | > 4,7 | > 4,7 |

**[Tableau 6]**

| Microorganismes | | *E. coli* | | *C. albicans* | | *A. brasiliensis* | | Acceptation à 28j |
|---|---|---|---|---|---|---|---|---|
| Composition | Temps (j) | 14 | 28 | 14 | 28 | 14 | 28 | |
| A | | > 4,7 | > 4,7 | 1,7 | 2,9 | -0,4 | -0,4 | Non conforme USP, EP |
| C | | > 4,7 | > 4,7 | > 4,7 | > 4,7 | 2 | 2 | Conforme USP et EP critère A |
| D | | > 4,7 | > 4,7 | > 4,7 | > 4,7 | 1 | 1 | Conforme USP et EP critère B |

La composition A, comprenant du méthyl parabène mais pas d'amitriptyline, n'est pas conforme aux tests de la pharmacopée européenne et américaine.

La composition C, comprenant du méthyl parabène et de l'amitriptyline, est conforme aux tests de la pharmacopée européenne et américaine. Il en est de même pour la composition D, comprenant de l'amitriptyline mais pas de méthyl parabène.

Ainsi, la présence d'amitriptyline permet de réduire les populations de microorganismes tels que *P. aeruginosa, S. aureus, E. coli, C. albicans et A. brasiliensis,* dans des compositions pharmaceutiques, même en absence d'un agent conservateur tel que le méthyl parabène.

## Revendications

1. Utilisation de l'amitriptyline et/ou l'un de ses sels pharmaceutiquement acceptables comme agent conservateur dans une composition pharmaceutique.

2. Utilisation de l'amitriptyline et/ou l'un de ses sels pharmaceutiquement acceptable selon la revendication 1, **caractérisée en ce que** la teneur totale en amitriptyline et/ou en l'un de ses sels pharmaceutiquement acceptables est comprise entre 1 et 25% en poids, de préférence entre 5 et 20% en poids, plus préférentiellement encore entre 5 et 15% en poids, mieux entre 10 et 15% en poids, par rapport au poids total de la composition.

3. Utilisation de l'amitriptyline et/ou l'un de ses sels pharmaceutiquement acceptable selon l'une quelconque des revendications précédentes, **caractérisée en ce que** la teneur en eau de la composition pharmaceutique est supérieure ou égale à 75% en poids, de préférence comprise entre 75 et 95% en poids ; et plus préférentiellement entre 75 et 90% en poids, par rapport au poids total de la composition.

4. Utilisation de l'amitriptyline et/ou l'un de ses sels pharmaceutiquement acceptable selon l'une quelconque des revendications précédentes, **caractérisée en ce que** le pH de la composition pharmaceutique est compris entre 3 et 8 ; de préférence entre 4 et 7 ; plus préférentiellement entre 5 et 6.

5. Utilisation de l'amitriptyline et/ou l'un de ses sels pharmaceutiquement acceptable selon l'une quelconque des revendications précédentes, **caractérisée en ce que** la composition comprend en outre au moins un polyol en C₂-C₈.

6. Utilisation de l'amitriptyline et/ou l'un de ses sels pharmaceutiquement acceptable selon l'une quelconque des revendications précédentes, **caractérisée en ce que** la composition comprend en outre au moins un agent épaississant ; de préférence choisi parmi les polymères cellulosiques.

7. Utilisation de l'amitriptyline et/ou l'un de ses sels pharmaceutiquement acceptable selon l'une quelconque des revendications précédentes, **caractérisée en ce que** la composition comprend en outre au moins un tensioactif choisi parmi les tensioactifs anioniques, les tensioactifs cationiques, les tensioactifs amphotères et/ou zwittérioniques, les tensioactifs non ioniques, et leurs mélanges, de préférence parmi les tensioactifs non ioniques et leurs mélanges.

8. Utilisation de l'amitriptyline et/ou l'un de ses sels pharmaceutiquement acceptables selon l'une quelconque des revendications précédentes, **caractérisée en ce que** la composition pharmaceutique est exempte d'agent conservateur additionnel ; de préférence la composition pharmaceutique est exempte de parabène.

9. Utilisation de l'amitriptyline et/ou l'un de ses sels pharmaceutiquement acceptables selon l'une quelconque des revendications précédentes, **caractérisée en ce que** la composition pharmaceutique est destinée à être appliquée par voie topique.
